# EUROPEAN PATENT APPLICATION

(11) **EP 2 286 810 A1**
(43) Date of publication of application: **23.02.2011**
(21) Application number: 09746676.7
(22) Date of filing: 15.05.2009
(51) Int. Cl.: A61K 31/4178, A61K 31/135, A61K 31/165, A61K 31/195, A61K 31/197, A61K 31/4525, A61K 31/55, A61K 31/5513, A61K 35/36, A61P 1/08, A61P 1/10, A61P 1/12, A61P 1/14, A61P 3/02

(54) **PHARMACEUTICAL COMPOSITION FOR TREATMENT OF FIBROMYALGIA**

(30) Priority: 16.05.2008 JP 2008130140
(71) Applicant: Axis, Inc., Kagoshima-shi Kagoshima 891-1305 (JP); Argenes, Inc., Tokyo 105-0001 (JP)
(72) Inventor: NISHIOKA Kusuki, Tokyo 150-0012 (JP); OKA Hiroshi, Yokohama-shi Kanagawa 225-0011 (JP); NOHARA Rieko, Tokyo 106-0045 (JP)
(74) Representative: Ellis, Katherine Elizabeth Sleigh
(86) International application number: PCT/JP2009/059075
(87) International publication number: WO 2009/139470

(57) **Abstract**

Disclosed is a pharmaceutical composition for the treatment of fibromyalgia, which comprises pilocarpine or a pharmacologically acceptable salt thereof. The pharmaceutical composition can be used as a novel therapeutic agent for fibromyalgia.

## Description

### TECHNICAL FIELD

The present invention relates to a pharmaceutical composition for the treatment of fibromyalgia or the like.

### BACKGROUND ART

Fibromyalgia syndrome (FMS) is a pain disease the cause of the disease is unknown, and there are about two million patients of FMS in Japan. The most remarkable feature of this sickness is that no abnormal symptom can be observed in a routine checkup despite the subjective symptoms such as pains. For this reason, it is difficult to make an established diagnosis, which may lead to a situation where either the treatment is impossible or the symptoms cannot be regarded as a sickness.

The diagnosis of fibromyalgia is made by the method with reference to the FMS classification criteria published in 1990 by the American College of Rheumatology (ACR) (Non-patent Document 1). To summarize, examples of the diagnostic points include: 1) persistence of systemic pain for 3 months or more; 2) no abnormal symptoms observed in a routine checkup; and 3) awareness of pain at 11 points or more, out of 18 predetermined tender points, when applied with a pressure of 4 kg or less. In addition, the disease conditions have also been evaluated using an index called Fibromyalgia Impact Questionnaire (FIQ).

Examples of the symptoms of fibromyalgia syndrome include a pain symptom, a physical symptom, a neurological symptom, and a psychological symptom. Specific aspects of the pain symptoms include a feeling of stiffness, pains in the joints and muscles, pains behind the eyes, pains in the oral cavity and pains in the head. In addition, examples of the physical symptoms include insomnia, a feeling of exhaustion, a feeling of fatigue, keratoconjunctivitis sicca (dry eye), xerostomia, frequent urination, diarrhea and constipation, enteritis, interstitial cystitis, dysmenorrhea and menstrual irregularities. Moreover, examples of the neurological symptoms include a limb sensory impairment, numbness, dizziness, tinnitus and gastric distress. Furthermore, examples of the psychological symptoms include a feeling of anxiety, a feeling of impatience, depression, sleep disorders, and impaired concentration and attention. As described above, the fibromyalgia syndrome is manifested by various symptoms other than the pains.

Examples of the therapeutic agents for fibromyalgia include an antidepressant, a tranquilizer, and an anti-inflammatory agent (painkiller). However, there has been room for improvements with these therapeutic agents in terms of therapeutic effects.

### Prior Art Documents

### Non-patent Documents

Non-patent Document 1: Wolfe F. et al., Arthritis Rheum., Feb. 1990, vol. 33(2), pp. 160-172.

### DISCLOSURE OF THE INVENTION

### PROBLEMS TO BE SOLVED BY THE INVENTION

Under such circumstances, there has been a demand for a novel therapeutic agent for fibromyalgia.

### MEANS FOR SOLVING THE PROBLEMS

The present invention is completed based on the discovery that a pharmaceutical composition which includes pilocarpine or a pharmacologically acceptable salt thereof is useful for the treatment of fibromyalgia or the like, as a result of conducting intensive studies in order to solve the above-mentioned problems.

That is, the present invention provides the following pharmaceutical composition for the treatment of fibromyalgia or the like.
(1) A pharmaceutical composition for the treatment of fibromyalgia which includes pilocarpine or a pharmacologically acceptable salt thereof.
(2) The composition according to the above (1), wherein the pharmacologically acceptable salt is pilocarpine hydrochloride.
(3) The composition according to the above (1) or (2), further including at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.
(4) Use of pilocarpine or a pharmacologically acceptable salt thereof in order to manufacture a pharmaceutical composition for the treatment of fibromyalgia.
(5) A pharmaceutical composition for the treatment of fibromyalgia which includes a combination of at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof, and pilocarpine or a pharmacologically acceptable salt thereof.
(6) A pharmaceutical composition for the treatment of fibromyalgia which includes pilocarpine or a pharmacologically acceptable salt thereof as an active ingredient which targets a patient receiving a composition including at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.
(7) A kit for the treatment of fibromyalgia which includes a composition including pilocarpine or a pharmacologically acceptable salt thereof as an active ingredient, and a composition including at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.
(8) A method for the treatment of fibromyalgia which includes administering pilocarpine or a pharmacologically acceptable salt thereof.
(9) The method according to the above (8), wherein the pharmacologically acceptable salt is pilocarpine hydrochloride.
(10) The method according to the above (8) or (9), further administering at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.

### EFFECTS OF THE INVENTION

According to the present invention, a pharmaceutical composition for the treatment of fibromyalgia or the like is provided. The composition or the like of the present invention is useful for the treatment of fibromyalgia or the like.

### BRIEF DESCRIPTION OF THE DRAWINGS

FIG. 1 is a questionnaire regarding the symptom of dry mouth (Example 2).
FIG. 2 is a questionnaire regarding the symptoms of dry eye, dry nose, dry skin and dry vagina (Example 2).

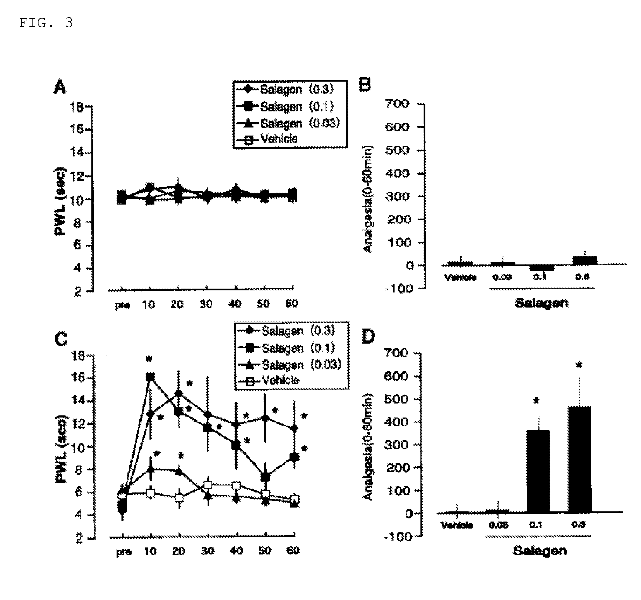

FIG. 3 is a graph showing the analgesic effect of pilocarpine hydrochloride (Example 3). The data are expressed as the mean ± S.E.M (standard error of the mean) (n = 4 to 9). * p < 0.05 (comparison with the vehicle group). A: A graph showing the changes in the analgesic effect of pilocarpine hydrochloride (Salagen (trade name)) (0.3, 0.1, 0.01 mg/kg, oral administration) over time in the control group (under non-stressed condition). B: A graph showing a dose-dependent analgesic effect of pilocarpine hydrochloride (Salagen (trade name)) (0.3, 0.1, 0.01 mg/kg, oral administration) in the control group (the data is expressed as the area under the curve (AUC)). C: A graph showing the changes in the analgesic effect of pilocarpine hydrochloride (Salagen (trade name)) (0.3, 0.1, 0.01 mg/kg, oral administration) over time in the intermittent cold stress (ICS) model group (under stressed condition). D: A graph showing a dose-dependent analgesic effect of pilocarpine hydrochloride (Salagen (trade name)) (0.3, 0.1, 0.01 mg/kg, oral administration) in the ICS model group (the data is expressed as the AUC).

### BEST MODE FOR CARRYING OUT THE INVENTION

The present invention will be described in detail below.
All the publications cited in the present specification, for example, prior art documents, laid-open patent applications, patent publications and other patent documents are incorporated herein by reference. In addition, the present specification incorporates the contents disclosed in the claims, specification and abstract of Japanese Patent Application No. 2008-130140, based on which priority is claimed.

### 1. Outline of the present invention

A pharmaceutical composition for the treatment of fibromyalgia of the present invention includes pilocarpine or a pharmacologically acceptable salt thereof. It is preferable that the pharmacologically acceptable salt be pilocarpine hydrochloride. It has been known that pilocarpine hydrochloride has an action to stimulate the muscarinic receptor and to improve the symptoms of xerostomia associated with the radiotherapy to the head and neck or xerostomia among Sjogren's syndrome patients. When the present inventors administered pilocarpine hydrochloride to the fibromyalgia patients, at least one symptom consisted of pains, xerostomia, keratoconjunctivitis sicca, interstitial cystitis, irritable bowel, or the like was alleviated. From this observation, it has become apparent that a composition which includes pilocarpine or a pharmacologically acceptable salt thereof is useful for the treatment of fibromyalgia. The composition of the present invention preferably includes, in addition to pilocarpine or a pharmacologically acceptable salt thereof, at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.

### 2. Fibromyalgia syndrome

Not only the onset mechanism of the fibromyalgia syndrome remains unknown but also the method for the treatment thereof has not been established. This is due to the fact that the fibromyalgia syndrome is manifested by various symptoms (such as physical symptoms, neurological symptoms and psychological symptoms) other than the pains. In other words, the fibromyalgia syndrome is a painful disorder of unknown cause and is manifested by a pain symptom, a physical symptom, a neurological symptom, and a psychological symptom. Specific examples of the pain symptoms include a feeling of stiffness, pains in the joints and muscles, pains behind the eyes, pains in the oral cavity and pains in the head. In addition, aspects of the physical symptoms include insomnia, a feeling of exhaustion, a feeling of fatigue, keratoconjunctivitis sicca (dry eye), xerostomia, frequent urination, diarrhea and constipation, enteritis, interstitial cystitis, dysmenorrhea and menstrual irregularities. Moreover, examples of the neurological symptoms include a limb sensory impairment, numbness, dizziness, tinnitus and gastric distress. Furthermore, examples of the psychological symptoms include a feeling of anxiety, a feeling of impatience, depression, sleep disorders, and impaired concentration and attention. As described above, the fibromyalgia syndrome is manifested by various symptoms other than the pains.

The diagnosis of fibromyalgia syndrome is made, for example, by the method with reference to the FMS classification criteria published in 1990 by the American College of Rheumatology (ACR) (Wolfe F. et al., Arthritis Rheum., Feb. 1990, vol. 33(2), pp. 160-172). More specifically, examples of the diagnostic points include: 1) persistence of systemic pain for 3 months or more; 2) no abnormal symptoms observed in a routine checkup; and 3) awareness of pain at 11 points or more, out of 18 predetermined tender points, when applied with a pressure of 4 kg or less.

### 3. Pilocarpine or a pharmacologically acceptable salt thereof

The chemical name for pilocarpine is (3S,4R)-3-ethyl-4-(1-methyl-1H-imidazol-5-ylmethyl)-4,5-dihydrofuran-2 (3H)-one. Although the pharmacologically acceptable salt of pilocarpine is not particularly limited, examples thereof include an inorganic acid salt (such as hydrochlorides or nitrates) and an organic acid salt (such as acetates or methanesulfonates). The pharmacologically acceptable salt of pilocarpine is preferably an inorganic acid salt (such as hydrochlorides or nitrates), and more preferably a hydrochloride.

Pilocarpine can be manufactured in accordance with a routine method. The method for manufacturing pilocarpine is described, for example, in "An improved synthesis of pilocarpine" (J. I. Degraw, Tetrahydron Vol. 28, Issue 4, 1972).

Pilocarpine as well as the pharmacologically acceptable salt thereof may be manufactured in accordance with a routine method or commercially available products thereof may be obtained. Examples of the commercially available products thereof include the pilocarpine hydrochlorides and pilocarpine nitrates available from Sigma-Aldrich, Inc.

The pilocarpine or the pharmacologically acceptable salt thereof obtained in this manner is used as an active ingredient in the pharmaceutical composition for the treatment of fibromyalgia of the present invention as described below.

### 4. Pharmaceutical composition

A pharmaceutical composition for the treatment of fibromyalgia of the present invention includes pilocarpine or a pharmacologically acceptable salt thereof. In addition, the present invention also includes the use of pilocarpine or a pharmacologically acceptable salt thereof in order to manufacture a pharmaceutical composition for the treatment of fibromyalgia. By administering the pharmaceutical composition according to the preferred embodiment of the present invention to the fibromyalgia syndrome patients, at least one symptom selected from the group consisting of pain symptoms (such as a feeling of stiffness, pains in the joints and muscles, pains behind the eyes, pains in the oral cavity and pains in the head), physical symptoms (such as insomnia, a feeling of exhaustion, a feeling of fatigue, keratoconjunctivitis sicca (dry eye), xerostomia, frequent urination, diarrhea and constipation, enteritis, interstitial cystitis, dysmenorrhea and menstrual irregularities, neurological symptoms (such as a limb sensory impairment, numbness, dizziness, tinnitus and gastric distress), and psychological symptoms (such as a feeling of anxiety, a feeling of impatience, depression, sleep disorders, and impaired concentration and attention) can be alleviated. More preferably, the pharmaceutical composition according to the preferred embodiment of the present invention can alleviate at least one symptom consisting of pains, xerostomia, keratoconjunctivitis sicca, interstitial cystitis, irritable bowel, or the like.

Although the dosage form of the pharmaceutical composition of the present invention may be either of oral administration and parenteral administration, it is preferably oral administration.

These dosage forms can be formulated in accordance with a routine method, and may also include a carrier or an additive which is pharmaceutically acceptable. Examples of such carriers and additives include water, a pharmaceutically acceptable organic solvent, collagen, polyvinyl alcohol, polyvinylpyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum aragic, casein, agar, polyethylene glycol, diglycerol, glycerol, propylene glycol, vaseline, paraffin, stearyl alcohol, stearic acid, human serum albumin, mannitol, sorbitol, lactose, and a surface active agent which is acceptable as a medicinal additive.

The above-mentioned additive is selected either alone or in an appropriate combination from the above examples depending on the dosage form of the pharmaceutical composition of the present invention. In the case of oral administration, the dosage form may be tablets, capsules, subtle granules, powders, granules, liquid medicines, syrups or the like, or an adequate formulation can be administered. In the case of parenteral administration, examples of the dosage form include injections. In the case of an injection, for example, a drug can be administered systemically or locally through an intravenous injection such as an intravenous drip.

A commercially available formulation can also be used as the composition which includes pilocarpine or a pharmaceutically acceptable salt thereof. Examples of the commercially available formulations include the Salagen (trade name) tablets (active ingredient: pilocarpine hydrochloride manufactured by Kissei Pharmaceutical Co., Ltd.).

The dose of pilocarpine or a pharmacologically acceptable salt thereof differs depending on the patient's age, sex and symptoms, and the route of administration, the frequency of administration and the dosage form. In the case of an adult (60 kg), for example, the dose per day may be from 0.1 mg to 1,000 mg, from 1 mg to 100 mg, or from 2.5 mg to 60 mg (for example, 2.5 mg, 5 mg, 10 mg, 15 mg, 20 mg 25 mg, 30 mg, 35 mg, 40 mg, 45 mg, 50 mg, 55 mg or 60 mg). The administration method is appropriately selected depending on the patient's age and symptoms. A drug may be administered, for example, once a day or may be divided into two to four doses per day.

In addition, the pharmaceutical composition of the present invention may also include, in addition to pilocarpine or a pharmacologically acceptable salt thereof, at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof. Here, the expression "include" means that at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof, and pilocarpine or a pharmacologically acceptable salt thereof are formulated into the same preparation.

Alternatively, the pharmaceutical composition of the present invention may be a pharmaceutical composition which includes a combination of at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof, and pilocarpine or a pharmacologically acceptable salt thereof. Here, the expression "includes a combination of" means that at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof, and pilocarpine or a pharmacologically acceptable salt thereof are formulated into separate preparations. In those cases where at least two components are selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof, there components may be formulated into the same preparation or may be formulated into separate preparations, but are preferably formulated into separate preparations.

Alternatively, the pharmaceutical composition of the present invention may be a pharmaceutical composition which includes pilocarpine or a pharmacologically acceptable salt thereof as an active ingredient which targets a patient receiving a composition including at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.

The chemical name for gabapentin is (1-aminomethylcyclohexyl) acetic acid. Although the pharmacologically acceptable salt of gabapentin is not particularly limited, examples thereof include an inorganic acid salt (such as hydrochlorides or nitrates), an inorganic basic salt (such as sodium salts, potassium salts or calcium salts) and an organic acid salt (such as acetates, benzoates, maleates, tartrates, fumarates or mesilates). Gabapentin as well as the pharmacologically acceptable salt thereof may be manufactured in accordance with a routine method or commercially available products thereof may be obtained. Examples of the commercially available products thereof include the Gabapen (trade name) tablets (active ingredient: gabapentin, manufactured by Pfizer Japan Inc.).

The chemical name for pregabalin is (S)-3-(aminomethyl)-5-methylhexanoic acid. Although the pharmacologically acceptable salt of pregabalin is not particularly limited, examples thereof include an inorganic acid salt (such as hydrochlorides or nitrates), an inorganic basic salt (such as sodium salts, potassium salts or calcium salts) and an organic acid salt (such as acetates, benzoates, maleates, tartrates, fumarates or mesilates). Pregabalin as well as the pharmacologically acceptable salt thereof may be manufactured in accordance with a routine method or commercially available products thereof may be obtained. Examples of the commercially available products thereof include the Lyrica (trade name) (active ingredient: pregabalin, manufactured by Pfizer Japan Inc.).

An extract from inflammatory cutaneous tissue of rabbits inoculated with vaccinia virus may be manufactured in accordance with a routine method or commercially available products thereof may be obtained. Examples of the commercially available products thereof include the Neurotropin (trade name) tablets (active ingredient: an extract from inflammatory cutaneous tissue of rabbits inoculated with vaccinia virus, manufactured by Nippon Zoki Pharmaceutical Co., Ltd.).

Clonazepam as well as the pharmacologically acceptable salt thereof may be manufactured in accordance with a routine method or commercially available products thereof may be obtained. Although the pharmacologically acceptable salt of clonazepam is not particularly limited, examples thereof include an inorganic acid salt (such as hydrochlorides or nitrates), an inorganic basic salt (such as sodium salts, potassium salts or calcium salts) and an organic acid salt (such as acetates, benzoates, maleates, tartrates, fumarates or mesilates). Examples of the commercially available products thereof include the Rivotril (trade name) (active ingredient: clonazepam, manufactured by Chugai Pharmaceutical Co., Ltd.).

Milnacipran may be manufactured in accordance with a routine method or commercially available products thereof may be obtained. Although the pharmacologically acceptable salt of milnacipran is not particularly limited, examples thereof include an inorganic acid salt (such as hydrochlorides or nitrates), an inorganic basic salt (such as sodium salts, potassium salts or calcium salts) and an organic acid salt (such as acetates, benzoates, maleates, tartrates, fumarates or mesilates), and more specific examples include milnacipran hydrochloride. Examples of the commercially available products thereof include the Toledomin (trade name) (active ingredient: milnacipran hydrochloride, manufactured by Asahi Kasei Pharma Corporation).

Paroxetine may be manufactured in accordance with a routine method or commercially available products thereof may be obtained. Although the pharmacologically acceptable salt of paroxetine is not particularly limited, examples thereof include an inorganic acid salt (such as hydrochlorides or nitrates), an inorganic basic salt (such as sodium salts, potassium salts or calcium salts) and an organic acid salt (such as acetates, benzoates, maleates, tartrates, fumarates or mesilates), and more specific examples include paroxetine hydrochloride. Examples of the commercially available products thereof include the Paxil (trade name) (active ingredient: paroxetine hydrochloride hydrate, manufactured by GlaxoSmithKline plc.).

Amitriptyline may be manufactured in accordance with a routine method or commercially available products thereof may be obtained. Although the pharmacologically acceptable salt of amitriptyline is not particularly limited, examples thereof include an inorganic acid salt (such as hydrochlorides or nitrates), an inorganic basic salt (such as sodium salts, potassium salts or calcium salts) and an organic acid salt (such as acetates, benzoates, maleates, tartrates, fumarates or mesilates), and more specific examples include amitriptyline hydrochloride. Examples of the commercially available products thereof include the Tryptanol (trade name) (active ingredient: amitriptyline hydrochloride, manufactured by Banyu Pharmaceutical Co., Ltd.).

Mianserin may be manufactured in accordance with a routine method or commercially available products thereof may be obtained. Although the pharmacologically acceptable salt of mianserin is not particularly limited, examples thereof include an inorganic acid salt (such as hydrochlorides or nitrates), an inorganic basic salt (such as sodium salts, potassium salts or calcium salts) and an organic acid salt (such as acetates, benzoates, maleates, tartrates, fumarates or mesilates), and more specific examples include mianserin hydrochloride. Examples of the commercially available products thereof include the Tetramide (trade name) tablets (active ingredient: mianserin hydrochloride, manufactured by Organon-Japan).

In those cases where gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof are used in combination, the dose thereof differs depending on the patient's age, sex and symptoms, and the route of administration, the frequency of administration and the dosage form. In the case of an adult (60 kg), for example, the dose of gabapentin or a pharmacologically acceptable salt thereof per day may be from 1 mg to 360 g, from 10 mg to 36 g or from 50 mg to 7,200 mg. In the case of an adult (60 kg), for example, the dose of pregabalin or a pharmacologically acceptable salt thereof per day may be from 0.2 mg to 60 g, from 2 mg to 6 g or from 10 mg to 1,200 mg. In the case of an adult (60 kg) and when the Neurotropin (trade name) tablets are used, for example, the dose of the extract from inflammatory cutaneous tissue of rabbits inoculated with vaccinia virus per day may be from 0.1 neurotropin units to 3,200 neurotropin units, from 1 neurotropin unit to 320 neurotropin units, or from 4 neurotropin units to 64 neurotropin units. In the case of an adult (60 kg), for example, the dose of clonazepam or a pharmacologically acceptable salt thereof per day may be from 0.005 mg to 600 mg, from 0.05 mg to 60 mg or from 0.25 mg to 12 mg. In the case of an adult (60 kg), for example, the dose of milnacipran or a pharmacologically acceptable salt thereof per day may be from 0.3 mg to 10 g, from 3 mg to 1 g or from 15 mg to 200 mg. In the case of an adult (60 kg), for example, the dose of paroxetine or a pharmacologically acceptable salt thereof per day may be from 0.1 mg to 4 g, from 1 mg to 400 mg or from 5 mg to 80 mg. In the case of an adult (60 kg), for example, the dose of amitriptyline or a pharmacologically acceptable salt thereof per day may be from 0.1 mg to 30 g, from 1 mg to 3 g or from 5 mg to 600 mg. In the case of an adult (60 kg), for example, the dose of mianserin or a pharmacologically acceptable salt thereof per day may be from 0.3 mg to 6 g, from 3 mg to 600 mg or from 15 mg to 120 mg. The administration method is appropriately selected depending on the patient's age and symptoms. A drug may be administered, for example, once a day or may be divided into two to four doses per day.

Although the dose of at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof is not particularly limited and differs depending on the individual combinations with pilocarpine or a pharmacologically acceptable salt thereof, for example, it is from about 0.001 to 10, 000 times (in terms of weight ratio) or from about 0.003 to 5,000 times (in terms of weight ratio), as much as that of pilocarpine or a pharmacologically acceptable salt thereof.

In those cases where at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof, and pilocarpine or a pharmacologically acceptable salt thereof are formulated into separate preparations, these preparations may be administered at the same time, or either one of them may be administered first followed by the other.

Examples of other drugs which can be used in combination with the pharmaceutical composition of the present invention include a drug which may be formulated for the treatment of fibromyalgia syndrome. Specific examples of such drugs include the Myslee (trade name) tablets (active ingredient: zolpidem tartrate, manufactured by Astellas Pharma Inc.), the Gaster (trade name) tablets (active ingredient: famotidine, manufactured by Astellas Pharma Inc.), and the Depas (trade name) tablets (active ingredient: etizolam, manufactured by Mitsubishi Tanabe Pharma Corporation).

### 5. Kit

A kit for the treatment of fibromyalgia of the present invention includes a composition including pilocarpine or a pharmacologically acceptable salt thereof as an active ingredient, and a composition including at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.
The kit of the present invention may also include a packaging container, an instruction manual, a package insert or the like, in addition to the above-mentioned compositions. In the packaging container, instruction manual, package insert or the like, the dosage and administration or the like for using the above-mentioned compositions in combination can be described. The dosage and administration can be described with reference to the explanation regarding the above-mentioned pharmaceutical compositions.

### 6. Treatment method

The present invention includes a method for the treatment of fibromyalgia which includes administering pilocarpine or a pharmacologically acceptable salt thereof. In this case, it is preferable that the pharmacologically acceptable salt be pilocarpine hydrochloride. Moreover, in addition to pilocarpine or a pharmacologically acceptable salt thereof, it is preferable to administer at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof. In the method for the treatment of fibromyalgia of the present invention, there are no particular limitations on the route of administration and the administration method for pilocarpine or a pharmacologically acceptable salt thereof, and the descriptions regarding the above-mentioned pharmaceutical composition can be referred to.

By applying the treatment method according to the preferred aspect of the present invention to the fibromyalgia syndrome patients, at least one symptom selected from the group consisting of pain symptoms (such as a feeling of stiffness, pains in the joints and muscles, pains behind the eyes, pains in the oral cavity and pains in the head), physical symptoms (such as insomnia, a feeling of exhaustion, a feeling of fatigue, keratoconjunctivitis sicca (dry eye), xerostomia, frequent urination, diarrhea and constipation, enteritis, interstitial cystitis, dysmenorrhea and menstrual irregularities, neurological symptoms (such as a limb sensory impairment, numbness, dizziness, tinnitus and gastric distress), and psychological symptoms (such as a feeling of anxiety, a feeling of impatience, depression, sleep disorders, and impaired concentration and attention) can be alleviated. More preferably, the treatment method according to the preferred aspect of the present invention can alleviate at least one symptom consisting of pains, xerostomia, keratoconjunctivitis sicca, interstitial cystitis, irritable bowel, or the like.

The present invention will be specifically described below using Examples. However, the present invention is not limited to these Examples.

### EXAMPLES

### [Example 1]

A Salagen tablet 5 mg (trade name) (active ingredient: pilocarpine hydrochloride) manufactured by Kissei Pharmaceutical Co., Ltd. was administered to 22 fibromyalgia syndrome patients (two males and 20 females) who were diagnosed based on the diagnostic criteria of the American College of Rheumatology (Wolfe F. et al., Arthritis Rheum., Feb. 1990, vol. 33(2), pp. 160-172) for a predetermined period of time to confirm the effects thereof. Examples of 4 patiens out of 22 patients who were administered with the drug will be specifically shown below.

### 1. Fibromyalgia syndrome patient A (52 years of age, female)

Prior to the administration of the Salagen tablet 5 mg (trade name), each symptoms of keratoconjunctivitis sicca, xerostomia, irritable bowel and interstitial cystitis was observed in the patient.
Two tablets of the Salagen tablet 5 mg (trade name) were administered to the patient per day (i.e., 10 mg/day). This patient also received the Toledomin tablet 25 (trade name), Gabapen tablet 200 mg (trade name), and Rivotril tablet 1 mg (trade name), in addition to the Salagen tablet 5 mg. The respective doses per day is as follows.
Toledomin tablet 25 (trade name) : 3 tablets per day (i.e., 75 mg/day)
Gabapen tablet 200 mg (trade name) : 5 tablets per day (i.e., 1,000 mg/day)
Rivotril tablet 1 mg (trade name) : 1 tablet per day (i.e., 1 mg/day)
When this patient was examined after 14 days from the start of administration of the Salagen tablet 5 mg (trade name), improvements were observed with respect to each symptom of xerostomia and irritable bowel. More specifically, the symptoms of xerostomia were improved and the food consumption became easier. In addition, the symptoms of irritable bowel were improved with fewer diarrheas.

### 2. Fibromyalgia syndrome patient B (52 years of age, female)

Prior to the administration of the Salagen tablet 5 mg (trade name), each symptoms of keratoconjunctivitis sicca, xerostomia and xeroderma was observed in this patient.
A half of the Salagen tablet 5 mg (trade name) was administered to this patient per day (i.e., 2.5 mg/day). This patient also received the Gabapen tablet 200 mg (trade name), Myslee tablet 5 mg (trade name), and Gaster tablet 10 mg (trade name), in addition to a half tablet of the Salagen tablet 5 mg (trade name) (i.e., 2.5 mg). The respective doses per day is as follows.
Gabapen tablet 200 mg (trade name): 3 tablets per day (i.e., 600 mg/day)
Myslee tablet 5 mg (trade name): 1 tablet per day (i.e., 5 mg/day)
Gaster tablet 10 mg (trade name) : 2 tablets per day (i.e., 20 mg/day)
When this patient was examined after 28 days from the start of administration of the Salagen tablet (trade name), improvements were observed with respect to each symptom of keratoconjunctivitis sicca, xerostomia and irritable bowel. More specifically, the symptoms of keratoconjunctivitis sicca were improved and a frequent instillation was reduced to once a day. Moreover, the symptoms of xerostomia were improved and saliva was produced so as to be dribbled out from the angle of the mouth. Furthermore, the symptoms of xeroderma were improved and the skin became moisturized, and thus it was no longer necessary to apply Hirudoid (trade name) to the skin.

### 3. Fibromyalgia syndrome patient C (71 years of age, female)

Prior to the administration of the Salagen tablet 5 mg (trade name), each symptom of systemic pains, keratoconjunctivitis sicca and xerostomia was observed in this patient.
One tablet of the Salagen tablet 5 mg (trade name) was administered to this patient per day (i.e., 5 mg/day).
When this patient was examined after 28 days from the start of administration of the Salagen tablet 5 mg (trade name), improvements were observed with respect to each symptom of keratoconjunctivitis sicca and xerostomia. On the other hand, the symptoms of systemic pains did not improve.

### 4. Fibromyalgia syndrome patient D (29 years of age, female)

Prior to the administration of the Salagen tablet 5 mg (trade name), in addition to the pain symptoms, each symptoms of keratoconjunctivitis sicca, xerostomia, irritable bowel and interstitial cystitis was observed in this patient.
Two tablets of the Salagen tablet 5 mg (trade name) were administered to this patient per day (i.e., 10 mg/day). This patient also received the Toledomin tablet 25 (trade name), Gabapen tablet 200 mg (trade name), and Depas tablet 0.5 mg (trade name), in addition to the Salagen tablet 5 mg (trade name). The respective doses per day is as follows.
Toledomin tablet 25 (trade name) : 2 tablets per day (i.e., 50 mg/day)
Gabapen tablet 300 mg (trade name): 4 tablets per day (i.e., 1,200 mg/day)
Depas tablet 0.5 mg (trade name) : 1 tablet per day (i.e., 0.5 mg/day)
When this patient was examined after 14 days from the start of administration of the Salagen tablet 5 mg (trade name), improvements were observed with respect to each symptom of keratoconjunctivitis sicca, xerostomia, irritable bowel and interstitial cystitis. More specifically, the symptoms of keratoconjunctivitis sicca were improved and the frequency of instillation was reduced. In addition, the symptoms of irritable bowel were improved with no abdominal pains. Further, the symptoms of interstitial cystitis were improved, the pains during urination were eliminated and the frequency of urination also reduced.

As described above, it became clear that by administering pilocarpine hydrochloride to the fibromyalgia syndrome patients, at least one symptom consisted of pains, xerostomia, keratoconjunctivitis sicca, interstitial cystitis or the like can be improved.

The summary of the 22 examples of patients (two males and 20 females) who were administered with the Salagen tablet 5 mg (trade name) is as follows.
Valid: 6 examples (4 examples out of these 6 examples are shown above)
Invalid and terminated: 7 examples
Terminated due to side effects: 2 examples (excessive sweating: 1 example, eruption: 1 example)
The effects currently being determined: 7 examples

### [Example 2]

A Salagen tablet 5 mg (trade name) (active ingredient: pilocarpine hydrochloride) manufactured by Kissei Pharmaceutical Co., Ltd. was administered to the following fibromyalgia syndrome patients who were diagnosed based on the diagnostic criteria of the American College of Rheumatology (Wolfe F. et al., Arthritis Rheum., Feb. 1990, vol. 33(2), pp. 160-172) for a predetermined period of time to confirm the effects thereof.

### Fibromyalgia syndrome patient E (54 years of age, female)

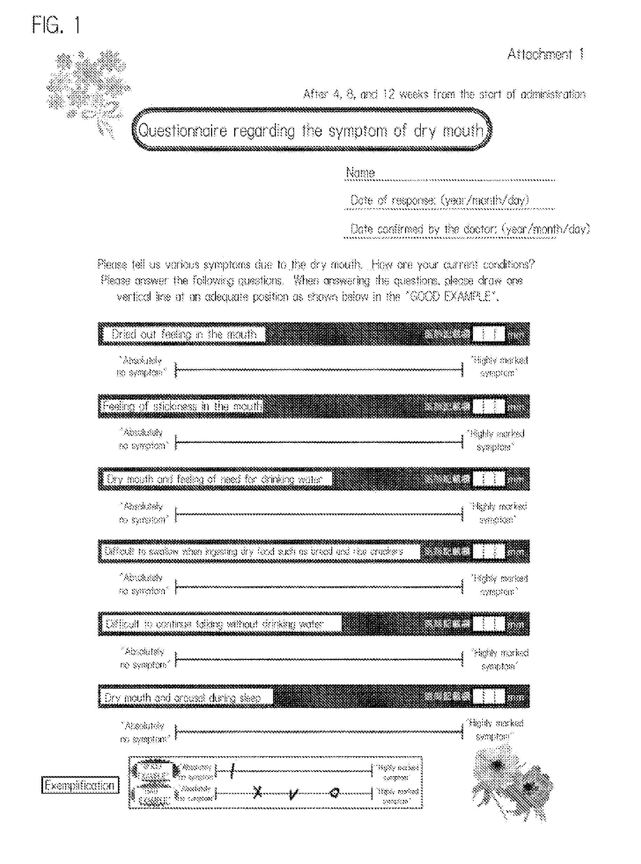

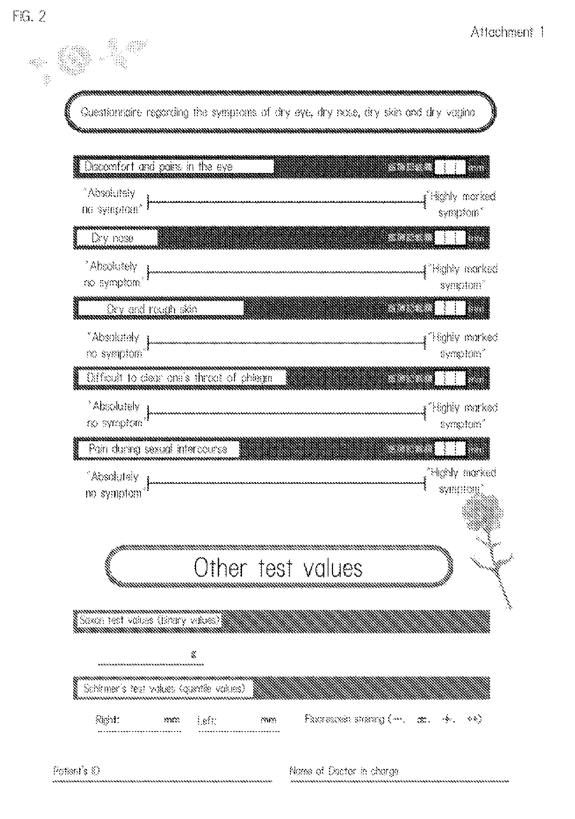

Prior to the administration of the Salagen tablet 5 mg (trade name), this patient answered each questions in the "questionnaire regarding the symptom of dry mouth" shown in FIG. 1 and the "questionnaire regarding the symptoms of dry eye, dry nose, dry skin and dry vagina" shown in FIG. 2. More specifically, the patient answered each questions by drawing a vertical line at the position indicating the current conditions on the line (length: 95 mm) drawn from "absolutely no symptom" to "highly marked symptom". After collecting the questionnaires, the length (mm) from the point "absolutely no symptom" up to the vertical line drawn at the position indicating the current condition was measured for each answers, and the measured results are shown in Table 1 shown below. As shown in Table 1, prior to the administration of the Salagen tablet 5 mg, in addition to the pain symptoms, each symptoms of keratoconjunctivitis sicca, xerostomia and xeroderma was observed in this patient.
Accordingly, two tablets of the Salagen tablet 5 mg (trade name) were administered to this patient per day (i.e., 10 mg/day). This patient also received the Depas (trade name), Mobic (trade name), Gabapen (trade name) and Neurotropin (trade name), in addition to the Salagen tablet 5 mg (trade name). The respective doses per day is as follows.
Depas (trade name): 1 tablet per day (i.e., 0.5 mg/day)
Mobic (trade name): 1 tablet per day (i.e., 10 mg/day)
Gabapen (trade name): 1 tablet per day (i.e., 300 mg/day)
Neurotropin (trade name): 4 tablets per day (i.e., 16 units/day)
After 7 days from the start of administration of the Salagen tablet 5 mg (trade name), this patient once again answered each questions in the "questionnaire regarding the symptom of dry mouth" shown in FIG. 1 and the "questionnaire regarding the symptoms of dry eye, dry nose, dry skin and dry vagina" shown in FIG. 2. The results of answers are also indicated in Table 1 shown below. In addition, in Table 1, the value determined by dividing the value obtained prior to the administration with the value obtained following the administration is also indicated as the rate of improvement. As shown in Table 1, when the conditions of the patient prior to and following the administration of the Salagen tablet 5 mg (trade name) were compared, improvements in each symptoms of keratoconjunctivitis sicca, xerostomia and xeroderma were observed.

**[Table 1]**

| Question | Before administration | After administration | Rate of improvement |
|---|---|---|---|
| Dried out feeling in the mouth | 83 | 47 | 1.77 |
| Feeling of stickiness in the mouth | 85 | 57 | 1.49 |
| Dry mouth and feeling of need for drinking water | 87 | 45 | 1.93 |
| Difficult to swallow when ingesting dry food such as bread and rice crackers | 88 | 63 | 1.40 |
| Difficult to continue talking without drinking water | 86 | 44 | 1.95 |
| Dry mouth and arousal during sleep | 70 | 40 | 1.75 |
| Discomfort and pains in the eye | 86 | 73 | 1.18 |
| Dry nose | 72 | 64 | 1.13 |
| Dry and rough skin | 73 | 72 | 1.01 |
| Difficult to clear one's throat of phlegm | 73 | 50 | 1.46 |

| | | | |
|---|---|---|---|
| "Absolutely no symptom"-"Highly marked symptom": 0 - 95 Unit: mm | | | |

### [Example 3]

### I. Method

### 1. Experimental animal and experimental environment

6 to 14-week old C57BL/6J male mice were used in all the experiments. The mice were fed in a room of constant temperature (24 ± 2°C) and constant humidity (55 ± 5%) with a 12 hr (from 8:00 to 20:00) light/dark cycle with a free access to the chow pellets (MF diet, Oriental Yeast Co., Ltd., Tokyo) for the standard experiment and drinking water.
In terms of the experimental environment, a room in the laboratory was used with a constant temperature (24 ± 2°C) and constant humidity (55 ± 5%). The mice used in the experiment were brought into the room by 24 hours prior to the start of the experiment and were fed until the experiment with a 12 hr (from 8:00 to 20:00) light/dark cycle with a free access to the chow pellets (MF diet, Oriental Yeast Co., Ltd., Tokyo) for the standard experiment and drinking water.
Further, the experiment was carried out between 10 a.m. and 5 p.m. The drug administration experiment was carried out between the period of time in which the symptoms of pain hypersensitivity due to the ICS stress stably persisted, from day 5 to day 14 following the stress termination.
All the experiments was carried out in accordance with both the guidelines for animal experiments in Nagasaki University and the method specified by the International Committee on the pain experiment (permission number for the animal experiment: 0706130596).

### 2. Drug administration

Tablets of pilocarpine hydrochloride (Salagen (trade name)) manufactured by Kissei Pharmaceutical Co., Ltd. were used. In addition, pilocarpine hydrochloride purchased from Wako Pure Chemical Industries, Ltd. was used. The respective drugs dissolved in sterile purified water were orally administered as a single dose. Three doses (i.e., concentrations of 0.03 mg/kg, 0.1 mg/kg and 0.3 mg/kg) were used. Sterile purified water was orally administered to the control group.
In the intraperitoneal administration and oral administration, 0.1 ml was administered for each 10 g of body weight.

### 3. Preparation of mouse model

### Intermittent cold stress (ICS) model

### (Chronic pain model due to the repeated cold stimulation)

The ICS model was used as an animal experiment model for fibromyalgia. In the present model, it has already been elucidated that prolonged hyperalgesia is developed following the stress, which is highly similar to the clinical symptoms in humans.
In terms of the breeding temperature for the ICS mouse model, a cycle of room temperature (i.e., 24°C) and low temperature (i.e., 4°C) was repeated during the daytime and adjusted to the low temperature during the nighttime. In terms of the breeding environment, in order to avoid moisture, another cage was placed upside down on top of the cage screen, and a gap was made between the cage and the screen using a piece of chow pellet (MF diet, Oriental Yeast Co., Ltd., Tokyo) for the standard experiment. In addition, sterile purified water was solidified using agar (manufactured by Asahi & Co., Ltd.) (1 g of agar for 100 ml of water) and the resultant was cut into 1 cm square pieces, and the mice were fed under the conditions of a constant humidity (55 ± 5%) with a 12 hr (from 8:00 to 20:00) light/dark cycle with a free access to the pieces of agar as the chow pellets and water.
At 16:30 on day 0, the mice used in the experiment were transferred to the inside of a fridge under the low temperature condition and were bred therein until the next day at 10:00.
The mice were transferred next day (day 1) at 10:00 to a room temperature condition, and thereafter bred until 16:30 under a low temperature condition for 30 minutes and a room temperature condition for 30 minutes, alternately.
The mice were also bred on day 2 in the same manner as that in day 1, and were transferred to a room temperature condition at 10:00 on day 3 to terminate the experiment.
The control group was bred under a room temperature condition throughout the three days.

### 4. Method for evaluating pain-associated behavior

Hargreaves test (thermal paw-withdrawal test) : pain testing method by thermal stimulation
A mouse to be used was placed in a plastic cage situated on top of a glass plate and left under the same environment for 30 minutes or more for acclimatization. A thermal stimulus was projected at the center of the hindlimb footpad from below the glass plate and the latency until the mouse exhibited an escape response in the hindlimb (i.e., Paw Withdrawal Latency (PWL)) was measured and evaluated. A stimulus was given in the experiment so that the latency of 10 to 12 seconds can be achieved in the normal animals, and the cut off time was set to 20 seconds in order to prevent the tissue damage.

### 5. Measurement of threshold value in the ICS mouse model and the effect of drug on the pain hypersensitivity

### I) Evaluation of pain hypersensitivity over time

Assay for the measurement of threshold value was carried out using the Hargreaves test. The day the ICS stress application started was defined as day 0, the day the ICS stress application terminated was defined as post stress day 1 (P1), and thereafter, the pain threshold values were measured until the P15. The latency and intensity were measured three times or more, and the mean was adopted. In addition, a time interval of 10 minutes was set until the next threshold value was measured. The reason for this time interval is to prevent the effects from the thermal stimulation measured before.

### II) Analgesic effect of pilocarpine hydrochloride

Evaluation was made by carrying out the measurement using the Hargreaves test. After a certain time period for adaptation, the drug solution prepared by dissolving pilocarpine hydrochloride tablets was orally administered as a single dose, and the latency until the escape response was exhibited was measured. The latency was measured every 10 minutes following the drug administration for 60 minutes. Sterile purified water was orally administered to the control group. The analgesic effect of pilocarpine hydrochloride was determined by calculating the area under the curve (AUC) from the response curve over time and subtracting the area under the curve when drawing a parallel line in the x-axis direction with respect to the threshold value prior to the drug administration.

### 6. Statistical processing

The data over time and the AUC of the analgesic effect were analyzed using the t-test for two independent groups. With respect to the AUC, the drug-administered group and the non-administered group were compared in the ICS stress mouse group. In terms of the statistics, the level of significance was set to 5% (indicated by the symbol *), and all the results are expressed as the mean ± S.E.M.
Sheffe's F test was used for the statistical analysis of the data over time. This is to carry out comparisons among all the independent multigroups, in which one-way analysis of variance is first performed, and if the result showed a significant difference among the groups, a multiple test is then used to determine among which groups the difference is present. This is comparing the analgesic effect by the dosage of individual pilocarpine hydrochloride tablets at the same time with that of the vehicle group. In terms of the statistics, the level of significance was set to 5% (indicated by the symbol *), and all the results are expressed as the mean ± S.E.M.

### II. Results

### 1. Variation in the pain threshold value due to the pilocarpine hydrochloride (Salagen) tablets

The drug efficacy of pilocarpine hydrochloride was assessed at three concentrations (i.e., 0.3 mg/kg, 0.1 mg/kg and 0.03 mg/kg) using a noxious heat test (FIG. 3).

There was no significant difference among all the groups before the start of the treatment, and the state of sensitivity was at the same level (i.e., control group: 5.6 ± 0.9 seconds, group administered with pilocarpine hydrochloride tablets (0.3 mg/kg): 4.2 ± 0.9 seconds, group administered with pilocarpine hydrochloride tablets (0.1 mg/kg) : 4.7 ± 0.4 seconds, and group administered with pilocarpine hydrochloride tablets (0.03 mg/kg): 5.9 ± 0.7 seconds) . However, the analgesic effect by the drug administration was confirmed due to the significant difference (FIG. 3C). The significant difference was also observed in the AUC results (i.e., control group: 8.0 ± 33.9, group administered with pilocarpine hydrochloride tablets (0.3 mg/kg): 460.2 ± 130.4, group administered with pilocarpine hydrochloride tablets (0.1 mg/kg) : 356.2 ± 59.2, and group administered with pilocarpine hydrochloride tablets (0.03 mg/kg) : 14.9 ± 38.3) (FIG. 3D). FIGS. 3A and 3B show the results of non-stressed group and no effect due to the drug administration at any dosage is observed.

In addition, the AUC for the analgesic effect increased in a dose-dependent manner (FIG. 3D). The analgesic effect persisted for 1 hour returned to the original value 3 hours after the administration (data not shown).

### 2. Analgesic effect of pilocarpine hydrochloride in the ICS mouse

The fibromyalgia syndrome patients tend to have an unbalanced autonomic nerve system which is caused by the hypertonic sympathetic nervous system or the like. As a result, the patients exhibit not only the systemic pains but also the symptoms similar to those of Sjogren's syndrome patients such as dry mouth. The pilocarpine hydrochloride used in this experiment is a non-selective muscarinic receptor agonist and is used for Sjogren's syndrome patients due to its effect to promote salivation. In this experiment, in order to further examine the analgesic effect of pilocarpine hydrochloride, the ICS model was used for evaluation. The results showed a significant difference, thereby indicating the improvements with respect to the pain hypersensitivity in the group orally administered with 0.3 mg/kg and 0.1 mg/kg of pilocarpine hydrochloride tablets in the Hargreaves test (FIG. 3).

In addition to the balance adjustments of autonomic nerve system due to the activation of the parasympathetic nerve serving as the site of action for the treatment, the action on the acetylcholine receptors in the brain and spinal cord is also possible. It has been elucidated in the animal experiments for some time that the muscarinic agonists or cholinesterase inhibitors administered into the spinal arachnoid space exhibit analgesic effects (Zhuo, M. and Gebhart, G. F., Tonoc cholinergic inhibition of spinal mechanical transmission, Pain Vol. 46 (1991) pp. 221-222; and Scatton, B., Dubois, A., Javoy-Agid, F. and Camus, A., Autoradiographic localication of musucarinc cholinergic receptors at various segmentral levels of the human spinal cord, neurosic. Lett. Vol. 49 (1984) pp. 239-245). The muscarinic receptors which are regarded as the site of action are concentrated in the second layer of the dorsal horn of the spinal cord (i.e., substantia gelatinosa) (Todd, A. J. and Sullican, A. C., Light microscope study of the coexistence of GABA-like and glycine-like immunoreactications in the spinal cord of the rat, J. Comp. Neurol., Vol. 296 (1990) pp. 496-505; Kumazawa, T. and Perl, E. R., Excitation of marginal and substatia gelationsa neurons in the primate spinal cord: indications of their place in dorsal horn functional orignization, J. Comp. Neurol., Vol. 177 (1978) pp. 417-434; and Yoshimura, M. and Jessell, T. M., Primary afferent-evoked synaptic responses and slow potential generation in rat substantia gelationsa neurons in vitro, J. Neurophysiol., Vol. 62 (1989) pp. 96-108), and since numerous cells containing GABA are present at this site (The role of supraspinal muscrinic receptor and GABAnergic system in neuropathic pain, PAIN RESEARCH Vol. 23 No. 2 (2008), the action particularly on the muscarinic receptors in the GABA neuron has been suggested. This is also suggested from the fact that carbachol which is a synthesized choline ester acts so as to facilitate the release of GABA. In addition, since the substantia gelatinosa is considered to be a site responsible for integrating the nociceptive information from the periphery, passing it onto the thalamic projection neurons, and controlling the output (Yoshimura, M. and Jessell, T. M., Primary afferent-evoked synaptic responses and slow potential generation in rat substantia gelationsa neurons in vitro, J. Neurophysiol., Vol. 62 (1989) pp. 96-108); and Facilitation of GABA Release by Carbachol and Neostigmine in Substatia Gelatinosa of Rat Spinal Cord. Pine research Vol. 12 (1997) pp. 65-72), its involvement in the transmission of pains has been suggested. From these reports, it is considered that increase in the level of GABA release due to the activation of the muscarinic receptors present in GABA and the activation of the GABA_{A} receptor are associated with the pain relief. In other words, it is possible that the opening of Cl-channel due to the GABA_{A} activation, followed by the hyperpolarization of membrane potential and the reduction of membrane resistance may be responsible for the stabilization of the membrane potential of the substantia gelatinosa neurons and the reduced level of excitability thereof. This also corresponds with the site of action for pilocarpine hydrochloride, and it is considered that the analgesic effect was manifested in this experiment due to these actions.

In addition, since it is possible that a cholinergic agent such as pilocarpine hydrochloride may act on the central nervous system in a dose-dependent manner (The role of supraspinal muscrinic receptor and GABAnergic system in neuropathic pain, PAIN RESEARCH Vol. 23, No. 2, 2008), the action thereof on the brain may also be possible. There is a study reporting the suppression of allodynia to the mechanical stimulation by the intracerebroventricular (i.c.v.) administration of a muscarinic receptor agonist McN-A-343 in the experiment using a neuropathic pain model, and the GABA_{B} receptor due to the activation of muscarinic M1 receptor in the brain is suggested as the site of action. Pilocarpine hydrochloride orally administered in this experiment may act both on the spinal cord and the brain, and it thus suggests the analgesic effect through the activation of GABA neurons.

An interesting discovery in this experiment was that the drug dosage which resulted in the improvements was similar to that actually used in the clinical practice (administration of 5 mg as one dose for an adult), and also this dosage did not show an analgesic effect on the normal mice. These findings suggest that the hyperalgesia observed in the sickness may be caused by the abnormalities in the parasympathetic nerves, on which pilocarpine acts.

## Claims

1. A pharmaceutical composition for the treatment of fibromyalgia which includes pilocarpine or a pharmacologically acceptable salt thereof.

2. The composition according to claim 1, wherein the pharmacologically acceptable salt is pilocarpine hydrochloride.

3. The composition according to claim 1 or 2, further including at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.

4. Use of pilocarpine or a pharmacologically acceptable salt thereof in order to manufacture a pharmaceutical composition for the treatment of fibromyalgia.

5. A pharmaceutical composition for the treatment of fibromyalgia which includes a combination of at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof, and pilocarpine or a pharmacologically acceptable salt thereof.

6. A pharmaceutical composition for the treatment of fibromyalgia which includes pilocarpine or a pharmacologically acceptable salt thereof as an active ingredient which targets a patient receiving a composition including at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.

7. A kit for the treatment of fibromyalgia which includes a composition including pilocarpine or a pharmacologically acceptable salt thereof as an active ingredient, and a composition including at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.

8. A method for the treatment of fibromyalgia which includes administering pilocarpine or a pharmacologically acceptable salt thereof.

9. The method according to the claim 8, wherein the pharmacologically acceptable salt is pilocarpine hydrochloride.

10. The method according to claim 8 or 9, further administering at least one component selected from the group consisting of gabapentin, pregabalin, an extract from inflammatory cutaneous tissue of rabbits inoculated with vaccinia virus, clonazepam, milnacipran, paroxetine, amitriptyline, mianserin, and a pharmacologically acceptable salt thereof.
